# EUROPEAN PATENT SPECIFICATION

(11) **EP 2 740 496 B1**
(45) Date of publication and mention of the grant of the patent: **13.09.2017**
(21) Application number: 12820503.6
(22) Date of filing: 02.08.2012
(51) Int. Cl.: A61L 15/00, A61L 27/00, A61L 31/04, A61P 41/00, A61L 31/14

(54) **ANTI-ADHESION MEMBRANE**
HAFTSCHUTZMEMBRAN
MEMBRANE ANTI-ADHÉSION

(30) Priority: 03.08.2011 JP 2011170439
(43) Date of publication of application: 11.06.2014
(73) Proprietor: Gunze Limited, Ayabe-shi, Kyoto 623-8511 (JP)
(72) Inventor: MATSUZAKI, Mitsutaka, Ayabe-shi Kyoto 623-8512 (JP); SATO, Hideki, Ayabe-shi Kyoto 623-8512 (JP)
(74) Representative: Adam, Holger
(86) International application number: PCT/JP2012/069703
(87) International publication number: WO 2013/018864

(56) References cited:
- EP-A1- 1 616 584
- WO-A1-98/16165
- WO-A1-2007/018093
- JP-A- 2006 006 448
- JP-A- 2006 006 448
- JP-A- 2007 075 595
- JP-A- 2008 012 216
- JP-A- 2010 104 785
- US-A1- 2010 114 313
- JOJI YOSHIZAWA ET AL.: 'Tokushu: Yaku ni Tatsu Zairyo, Kigu, Sogu Arekore Shoni Geka Shujutsu ni Yakudatsu Zairyo, Kigu, Sochi - Yuchaku Boshi Zairyo' JAPANESE JOURNAL OF PEDIATRIC SURGERY vol. 36, no. 8, 2004, pages 1029 - 1033, XP008173611

## Description

### TECHNICAL FIELD

The present invention relates to an anti-adhesion membrane that can be readily rolled up into a thin tube and introduced into the body through a trocar without damage, and has moderate stiffness, and therefore is easy to handle.

### BACKGROUND ART

Adhesions frequently form between tissues in the body after surgery, and can be a cause of pain and dysfunction. Adhesion formation can be a serious problem, in particular, in the fields of obstetrics and gynecology, digestive surgery, orthopedics, and cardiovascular surgery. Some serious cases require surgery to detach adhesions. In case of reoperation for a primary disease, adhesions add risk. One known strategy to prevent adhesions between tissues in the body is to separate areas at risk of adhesion formation with a membrane called "anti-adhesion membrane".

Anti-adhesion membranes are required to have the following characteristics, for example: these water-containing gels are flexible enough to fit affected areas, can maintain their shape in the body for a certain period of time and then are absorbed immediately in the body, and cause less tissue reaction. Anti-adhesion membranes that meet these requirements are disclosed in, for example, Patent Literatures 1 to 6. As examples of commercially available anti-adhesion membranes, there may be mentioned films containing hyaluronic acid and carboxymethyl cellulose.

A recent trend has been towards reducing the burden on patients as much as possible. This has largely increased the number of endoscopic surgeries. This also has promoted the use of an instrument called "trocar" to apply an anti-adhesion membrane for preventing adhesion formation between tissues in the body. An anti-adhesion membrane is passed through this thin tube, and introduced into the body.

Unfortunately, conventional commercial anti-adhesion membranes including a film containing hyaluronic acid and carboxymethyl cellulose may be broken when rolled up into thin tubes because they are hard and brittle. Or, even if they can be rolled up, they may be broken while being passed through a trocar.

In order to solve this problem, an attempt to provide flexibility by reducing the thickness of anti-adhesion membranes was made, as in JP 2006-006448A. This attempt, however, resulted in the problems that thinner films have reduced strength and fail to have sufficient stiffness, and therefore get wrinkled when rolled up into thin tubes; and they easily deform due to static electricity, and therefore are very difficult to handle.

### CITATION LIST

### - Patent Literature

- Patent Literature 1:: JP H11-47258 A
- Patent Literature 2:: JP H11-279296 A
- Patent Literature 3:: JP 2000-212286 A
- Patent Literature 4:: JP 2003-62063 A
- Patent Literature 5:: JP 2004-209228 A
- Patent Literature 6:: Japanese Patent No. 3517358

### SUMMARY OF INVENTION

### - Technical Problem

An object of the present invention is to provide an anti-adhesion membrane that can be readily rolled up into a thin tube and introduced into the body through a trocar without damage, and has moderate stiffness, and therefore is easy to handle.

### - Solution to Problem

The present invention provides an anti-adhesion membrane including a film made of a bioabsorbable material, the film being provided with projections and recesses, as defined by claim 1. The following description is offered to illustrate the present invention in detail.

Intensive studies by the present inventors have revealed that by forming projections and recesses on anti-adhesion membranes including a film made of a bioabsorbable material, flexibility can be secured even when the thickness is enough to secure sufficient stiffness, and such films can be rolled up into thin tubes and introduced into the body through a trocar without damage. Thus, the present invention was completed.

The anti-adhesion membrane of the present invention includes a film made of a bioabsorbable material.

The bioabsorbable material for the film is not limited at all, and examples include synthetic absorbable polymers such as polyglycolide, polylactide (D, L, DL isomer), polycaprolactone, glycolide-lactide (D, L, DL isomer) copolymers, glycolide-ε-caprolactone copolymers, lactide (D, L, DL isomer)-ε-caprolactone copolymers, poly(p-dioxanone), and glycolide-lactide(D, L, DL isomer)-ε-caprolactone copolymers; and natural polymers such as gelatin, collagen, fibrin, hyaluronic acid, and carboxymethyl cellulose. Any of these may be used alone, or two or more of these may be used in combination. In particular, the combination of carboxymethyl cellulose and one of gelatin and hyaluronic acid is preferable because these combinations provide flexibility that allows the water-containing gel to fit an affected area, and enable the gel to maintain its shape in the body for a certain period of time and then to be absorbed immediately in the body.

In the case where the bioabsorbable material contains gelatin, any gelatin raw material can be used without limitation. For example, gelatin derived from bones, tendon, or skin of bovine, swine, chicken, or salmon can be used.

The weight average molecular weight, as determined by GPC, of the gelatin raw material for the gelatin film preferably has a lower limit of 100,000, and an upper limit of 300,000. If the weight average molecular weight is less than 100,000, the anti-adhesion membrane of the present invention may have low tensile strength. Gelatin does not have a molecular weight over 300,000 in nature. This value is set as the upper limit. The more prefrable lower limit of the weight average molecular weight is 150,000.

The jelly strength of the gelatin raw material preferably has a lower limit of 150 g, and an upper limit of 350 g. These limits are determined because: the anti-adhesion membrane of the present invention may have a large rate of deformation or low tensile strength when gelatin with a jelly strength of less than 150 g is used; and gelatin with a jelly strength of more than 350 g is difficult to produce. The more preferable lower limit of the jelly strength is 250 g.

The "jelly strength" herein refers to the jelly strength specified in JIS K 6503-2001. This strength is determined by: preparing a jelly by cooling a 6.67% by weight gelatin aqueous solution at 10°C for 17 hours; forcing a 12.7 mm-diameter plunger against it; and reading a force necessary to depress the jelly surface by 4 mm at 1 mm/sec.

The gelatin raw material is preferably alkali-treated gelatin. Such gelatin contains very little endotoxin, and has a high safety profile. Specific examples include alkali-treated bovine gelatin and alkali-treated swine gelatin available from Nippi Incorporated.

The gelatin raw material may contain known additives, as long as they don't adversely affect the object of the present invention. Examples of additives include additives for providing flexibility to the film, such as glycerin, polyethylene glycol, and hyaluronic acid. Other known additives, such as antimicrobial agents and antiinflammatory drugs, may be contained.

In the case where the bioabsorbable material contains gelatin, the gelatin is preferably crosslinked. The use of crosslinked gelatin prevents the anti-adhesion membrane from absorbing water and swelling so much, or degrading to lose its shape in a short time when the membrane is embedded in the body.

The gelatin can be crosslinked by any method without limitation, and conventionally known methods, such as thermal crosslinking, ultraviolet crosslinking, and chemical crosslinking using a crosslinking agent can be used. In particular, thermal crosslinking and ultraviolet crosslinking are preferred because they can form evenly distributed crosslinks with no risk of leaving crosslinking agents. More preferred is thermal crosslinking.

In the case where the bioabsorbable material contains hyaluronic acid and carboxymethyl cellulose, the hyaluronic acid has a viscosity of 30 to 150 mPa·s when measured in the form of a 1% by weight aqueous solution, and the carboxymethyl cellulose has a viscosity of 2 to 50 mPa·s when measured in the form of a 0.5% by weight aqueous solution. Hyaluronic acid and carboxymethyl cellulose having molecular weights within ranges that correspond to these viscosity ranges facilitate film formation, and the resulting film is flexible enough to fit an affected area, and can maintain its shape for a certain period of time in the body, and then is absorbed immediately in the body.

The film made of the bioabsorbable material is provided with projections and recesses. The projections and recesses contribute to flexibility of the anti-adhesion membrane, which is better than that of flat films made of the same bioabsorbable material and having the same thickness. Accordingly, such a film is readily rolled up into a thin tube and passed through a trocar without damage. Additionally, the projections and recesses enable ensure moderate stiffness and ease of handling.

The expression "film provided with projections and recesses" herein refers to a film provided with both rounded projections and recesses on a surface thereof, and therefore such a film is clearly different from films obtained by embossing flat films (by forming projections).

The shape of projections and recesses is not limited at all. For example, projections and recesses of a pin spot pattern, ruminating pattern, or pyramid pattern may be mentioned. In particular, projections and recesses of a pin spot pattern are preferable.

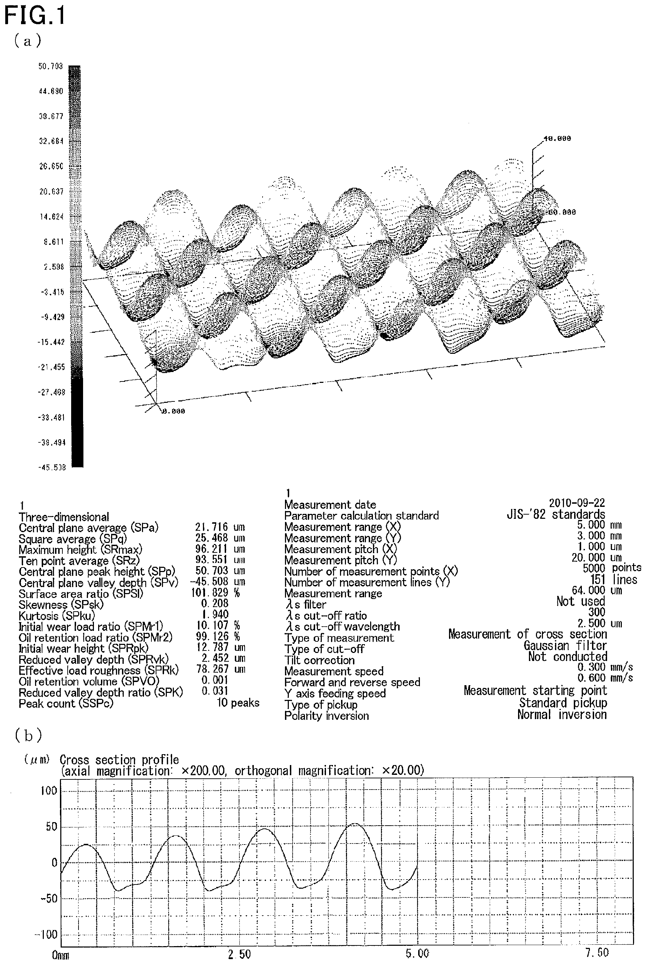

The term "pin spot pattern" herein refers to a pattern in which rounded projections and rounded recesses are arranged in a regular pattern. Figs. 1 show a graphical depiction of a three-dimensional analysis of projections and recesses of a pin spot pattern on a surface of an anti-adhesion membrane that was actually formed by the present inventors (Fig. 1(a)), and a cross section of the anti-adhesion membrane drawn based on the graphical depiction of the three-dimensional analysis (Fig. 1(b)).

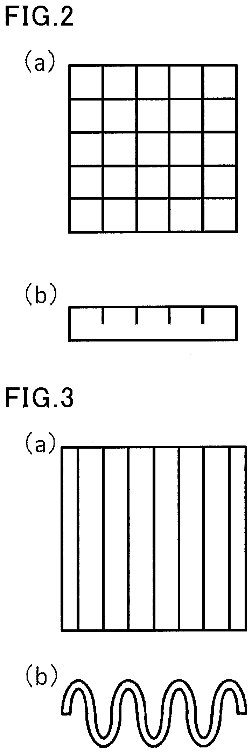

The term "lattice pattern" herein refers to a pattern in which flat-top projections and recesses are arranged in a regular pattern. Figs. 2 show a schematic diagram illustrating a surface of an anti-adhesion membrane with projections and recesses of a lattice pattern of the present invention (Fig. 2(a)), and a schematic cross-section thereof (Fig. 2(b)).

The term "ruminating pattern" herein refers to a ripple pattern in which continuous projected lines and continuous recessed lines alternately run in parallel. Figs. 3 show a schematic diagram illustrating a surface of an anti-adhesion membrane with projections and recesses of a ruminating pattern of the present invention (Fig. 3(a)), and a schematic cross section thereof (Fig. 3(b)).

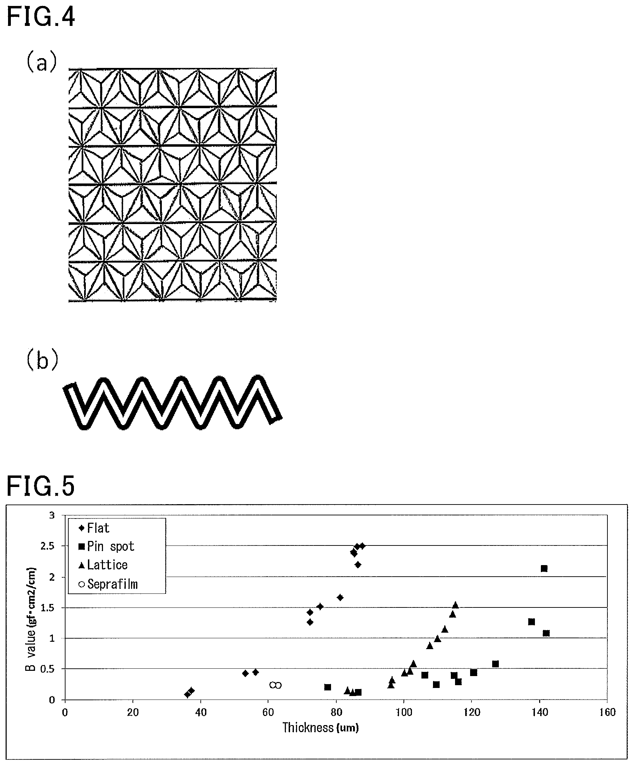

The term "pyramid pattern" herein refers to a pattern in which projections with a multi-sided pyramid shape and recesses with a multi-sided pyramid shape are arranged in a regular pattern. Figs. 4 are a schematic diagram illustrating a surface of an anti-adhesion membrane with projections and recesses of a pyramid pattern of the present invention (Fig. 4(a)), and a schematic cross section thereof (Fig. 4(b)).

The height difference between projections and recesses, or the distance between the top of projections and the bottom of recesses are not limited at all, and can be appropriately determined according to their shapes. In the case of, for example, projections and recesses of a pin spot pattern, the height difference between projections and recesses preferably has a lower limit of 30 µm, and an upper limit of 200 µm. If the height difference between projections and recesses is less than 30 µm, the projections and recesses are so small that the film has excessive stiffness which is attributed to its thickness. Accordingly, the membrane may be difficult to roll up or may be damaged when rolled up into a thin tube, and passed through a trocar. Designs with a height difference between projections and recesses of more than 200 µm are not suitable for industrial production. Specifically, in the process of casting a solution into an anti-adhesion membrane of the present invention by a casting method described below, a large amount of solution is needed to reach a level higher than projections of a release sheet. Unfortunately, it takes a very long time to dry the solution. The more preferable lower limit of the height difference between projections and recesses is 50 µm, and the more preferable upper limit is 180 µm.

The pitch of projections and recesses, in other words, the distance between the peaks of adjacent projections is not limited at all, and can be appropriately determined according to the shape. In the case of, for example, projections and recesses of a pin spot pattern, the preferable lower limit of the pitch is 10 µm, and the preferable upper limit is 20000 µm. If the pitch is designed to be less than 10 µm, it is difficult to produce anti-adhesion membranes with projections and recesses having a desired height difference. If the pitch is more than 20000 µm, the anti-adhesion membrane may not have sufficient stiffness, and may be difficult to handle. The more preferable lower limit of the pitch is 20 µm, and the more preferable upper limit 10000 µm.

The thickness of the film made of the bioabsorbable material, in other words, the distance between the peaks of projections on the respective surfaces is not limited at all. The preferable lower limit is 30 µm, and the preferable upper limit is 200 µm. If the thickness of the film made of the bioabsorbable material is less than 30 µm, the film made of the bioabsorbable material may not have sufficient stiffness, and may be difficult to handle. If the thickness is more than 200 µm, the membrane may be difficult to roll up or may be damaged when rolled up into a thin tube, and passed through a trocar. The more preferable lower limit of the thickness is 100 µm, and the more preferable upper limit is 120 µm.

The anti-adhesion membrane of the present invention can be produced by any method without limitation, and specifically can be produced by: dissolving the bioabsorbable material, which is a raw material for the membrane, in an appropriate solvent; casting the resulting solution onto a release sheet subjected to a water-repelling treatment (e.g. a glass plate, a polystyrene sheet (tray), or a fluororesin sheet (tray)); and drying the solution (cast method). A release sheet already having a desired projection and recess pattern can be used to provide a film with projections and recesses.

In the case where the bioabsorbable material contains gelatin, hyaluronic acid, and carboxymethyl cellulose, distilled water, dimethyl sulfoxide (DMSO), and mixtures of these can be used as a solvent for dissolving these materials, for example. Among these, preferred is distilled water for reasons of ease of handling.

The amount of the bioabsorbable material used is not limited at all. The preferable lower limit is 0.1 g per 100 mL of the solvent, and the preferable upper limit is 50 g. If the amount is less than 0.1 g, it is difficult to form a film. If the amount is more than 50 g, the solution is too viscous, and may be difficult to uniformly cast. The more preferable lower limit is 1 g, and the more preferable upper limit is 30 g.

The dissolving temperature is not limited at all. The preferable lower limit is 30°C, and the preferable upper limit is 70°C. At a temperature of lower than 30°C, it may take too long a time to dissolve the materials. At a temperature of higher than 70°C, gelatin may degrade into smaller molecules, which leads to reduced jelly strength. The more preferable lower limit is 40°C, and the more preferable upper limit is 60°C.

The drying can be accomplished by any method without limitation, and examples include air drying, heated-air drying, reduced-pressure drying (vacuum drying), forced draft drying, and forced air-circulation/forced convection drying.

The preferable lower limit of the drying temperature is -40°C, and the preferable upper limit is 60°C. At a temperature of lower than -40°C, the drying may take an undesirably long time, and at a temperature of higher than 60°C, gelatin may degrade into smaller molecules. The more preferable lower limit is 0°C, and the preferable upper limit is 40°C.

It is preferable that the above-mentioned procedures for producing a film made of a bioabsorbable material be aseptically carried out in, for example, a clean bench or a clean room. This is because the bioabsorbable material should be protected from bacterial growth and contamination in the process of production. Accordingly, it is preferable to use tools and implements sterilized, for example, in an autoclave or with EOG (gaseous ethylene oxide), hot air, or electric beams. Likewise, the gelatin solution is preferably subjected to sterilization by filter filtration in a known manner before the above-mentioned procedures.

The film made of the bioabsorbable material thus obtained is not crosslinked yet. The film is optionally crosslinked by, for example, heating or ultraviolet radiation.

The ultraviolet crosslinking is not limited at all, and it is preferable to irradiate both surfaces of the film with ultraviolet rays from an ultraviolet radiator.

The thermal crosslinking is not limited at all, and it is preferable to uniformly heat both surfaces of the film. Heating both surfaces of the film produces crosslinks evenly distributed in the thickness direction as well.

The heating is preferably carried out at a reduced pressure of not higher than 1 Torr. Under a reduced pressure, degradation of the gelatin due to heat can be reduced.

### - Advantageous Effects of Invention

The present invention provides an anti-adhesion membrane that can be readily rolled up into a thin tube and introduced into the body through a trocar without damage, and has moderate stiffness, and therefore is easy to handle.

### BRIEF DESCRIPTION OF DRAWINGS

[Fig. 1] Fig. 1(a) is a graphical depiction of a three-dimensional analysis of projections and recesses of a pin spot pattern on a surface of an anti-adhesion membrane of the present invention, and Fig. 1(b) is a cross section of the anti-adhesion membrane drawn based on the graphical depiction of the three-dimensional analysis.
[Fig. 2] Fig. 2(a) is a schematic diagram illustrating a surface of an anti-adhesion membrane with projections and recesses of a lattice pattern of the present invention, and Fig. 2(b) is a schematic cross-section thereof.
[Fig. 3] Fig. 3(a) is a schematic diagram illustrating a surface of an anti-adhesion membrane with projections and recesses of a ruminating pattern of the present invention, and Fig. 3(b) is a schematic cross section thereof.
[Fig. 4] Fig. 4(a) is a schematic diagram illustrating a surface of an anti-adhesion membrane with projections and recesses of a pyramid pattern of the present invention, and Fig. 4(b) is a schematic cross section thereof.
[Fig. 5] Fig. 5 is a graph of the bending rigidity (B values) of anti-adhesion membranes obtained in Experiment 1.
[Fig. 6] Fig. 6 is a graph of the bending hysteresis (2HB values) of the anti-adhesion membranes obtained in Experiment 1.
[Fig. 7] Fig. 7 is a graph of the static friction of the anti-adhesion membranes obtained in Experiment 1.
[Fig. 8] Fig. 8 is a graph of the bending rigidity (B values) of anti-adhesion membranes obtained in Experiment 2.
[Fig. 9] Fig. 9 is a graph of the bending hysteresis (2HB values) of the anti-adhesion membranes obtained in Experiment 2.

### DESCRIPTION OF EMBODIMENTS

The following describes examples to more specifically illustrate embodiments of the present invention. It should be noted that the present invention is not limited only to these examples.

### (Experiment 1)

Gelatin (Nippi Incorporated, alkali-treated swine gelatin, weight average molecular weight: 132000, jelly strength: 257 g) was dissolved in distilled water to a 5% by weight solution, and a 10 mL portion of this solution was cast onto a plastic release sheet with projections and recesses on the surface (size: 10 cm × 10 cm), and air-dried into an uncrosslinked gelatin film.

In the same way, uncrosslinked gelatin films of a pin spot pattern, uncrosslinked gelatin films of a lattice pattern, uncrosslinked gelatin films of a ruminating pattern, uncrosslinked gelatin films of a pyramid pattern, and flat uncrosslinked gelatin films without projections and recesses having various thicknesses were obtained by using release sheets with an appropriate surface pattern and changing the amount of the gelatin solution.

The uncrosslinked gelatin films were each sandwiched between 1 mm thick fluororesin sheets, and the resulting laminates were further sandwiched between 3 mm thick aluminum plates. The resulting laminates were placed on a shelf in a vacuum drier, and thermally crosslinked at 135°C under a pressure of 1 Torr or lower for 8 hours. In this manner, anti-adhesion membranes were obtained.

Figs. 1 show a graphical depiction of a three-dimensional analysis of an anti-adhesion membrane obtained above. The membrane included a crosslinked gelatin film with projections and recesses of a pin spot pattern. As shown in Figs. 1, the pitch of projections and recesses, that is, the distance between the peaks of adjacent projections of the crosslinked gelatin films with projections and recesses of a pin spot pattern was 1250 µm. The film thickness, that is, the distance between the peaks of projections and the bottoms of recesses was controlled within the range of 77 to 150 µm by adjusting the amount of the gelatin used.

Figs. 2 shows schematic diagrams of an anti-adhesion membrane including a crosslinked gelatin film with projections and recesses of a lattice pattern. The pitch of projections and recesses, that is, the distance between grid lines of the crosslinked gelatin films with projections and recesses of a lattice pattern was 1200 µm, and the depth of recesses was 30 µm. The film thickness was controlled within the range of 83 to 115 µm by adjusting the amount of the gelatin used.

Figs. 3 show schematic diagrams of an anti-adhesion membrane including a crosslinked gelatin film with projections and recesses of a ruminating pattern. The pitch of projections and recesses, that is, the distance between projections of the crosslinked gelatin films with projections and recesses of a ruminating pattern was 4500 µm. The film thickness, that is, the distance between the peaks of projections and the bottoms of recesses was 1000 µm.

Figs. 4 show schematic diagrams of an anti-adhesion membrane including a crosslinked gelatin film with projections and recesses of a pyramid pattern. The crosslinked gelatin films with projections and recesses of a pyramid pattern had projections and recesses arranged in a regular pattern, and the projections and recesses each had a regular triangular pyramid shape, each side of which is 5 mm long. The pitch of projections and recesses, that is, the distance between the peaks of adjacent projections was 3 mm, and the thickness, that is, the distance between the peaks of projections and the bottoms of recesses was 150 µm.

### (Evaluation)

The anti-adhesion membranes obtained above were evaluated as follows. Commercial anti-adhesion membranes ("Seprafilm" available from Genzyme Corporation), which include a film made of hyaluronic acid and carboxymethyl cellulose, were evaluated as controls in the same manner.

Seprafilm membranes are flat membranes without projections and recesses.

### (1) Evaluation of bending rigidity and bending hysteresis

The anti-adhesion membranes were evaluated for bending rigidity and bending hysteresis by a KES pure bending test. Specifically, each film was bent from its horizontal state (origin) upward to a certain level, and then returned to its horizontal state, and bent downward to a certain level, and then returned to its horizontal state. Their B value and 2HB value were determined by measuring the forces that occurred in this series of procedures.

The B value indicates the stiffness of a film measured while the film is bent by an external force. A larger B value indicates that a larger force is required to bend this film. Fig. 5 shows the results of the B value measurement on the anti-adhesion membranes.

As seen in Fig. 5, the comparison of membranes with the same thickness revealed that anti-adhesion membranes with projections and recesses have a smaller B value than flat anti-adhesion membranes, that is, are bent more softly. The comparison of membranes with the same B value revealed that anti-adhesion membranes with projections and recesses are about twice as thick as flat membranes with the same B value.

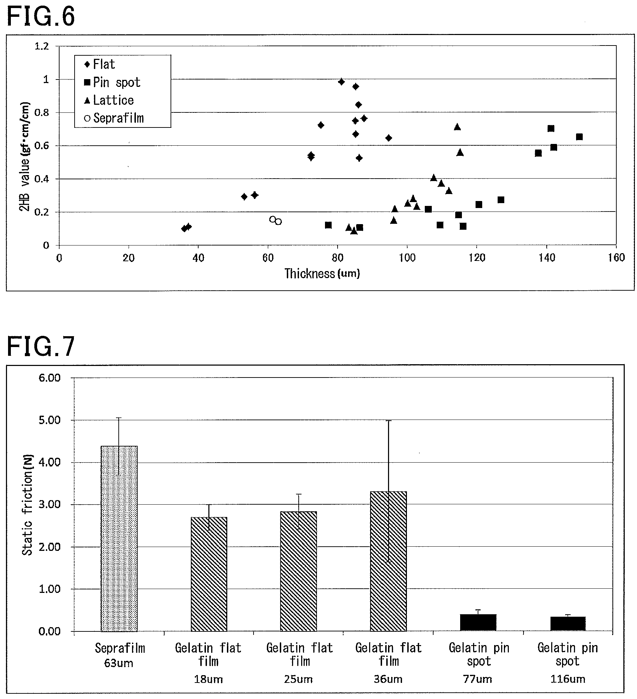

The 2HB value indicates the resilience of a film measured while the film is bent by an external force. A larger 2HB value corresponds to higher ability to return the film to the original shape. Fig. 6 shows the results of the 2HB value measurement on the anti-adhesion membranes.

As seen in Fig. 6, the comparison of membranes with the same thickness revealed that anti-adhesion membranes with projections and recesses have a smaller 2HB value than flat anti-adhesion membranes, that is, are less likely to return to the original shape. Accordingly, anti-adhesion membranes with projections and recesses can be more readily rolled up into thin tubes. The comparison of membranes with the same 2HB value revealed that anti-adhesion membranes with projections and recesses are about twice as thick as flat membranes with the same 2HB value.

### (2) Evaluation of minimum R value

The anti-adhesion membranes were rolled on tubes with different curvature radii R (mm) around their longitudinal axis. The minimum R value of each film was the curvature radius of the smallest tube on which the film could be rolled without damage. Namely, a smaller minimum R value indicates that a film can be rolled up into a thinner tube.

Table 1 shows the results.

**[Table 1]**

| Shape of film | Thickness (µm) | Minimum R (mm) |
|---|---|---|
| Projection and recesses (pin spot pattern) | 101 | 2.5 |
| Flat film | 101 | 3.9 |
| Seprafilm | | 5.8 |

As seen in Table 1, the comparison of membranes with the same thickness revealed that anti-adhesion membranes with projections and recesses have a smaller minimum R value than flat anti-adhesion membranes, that is, can be rolled up into thinner tubes.

### (3) Evaluation of friction resistance

The anti-adhesion membranes were each attached to a silicone mat so that a 1-cm² area was in contact with the mat. One end of each membrane was fixed with clamps, and the membranes were pulled horizontally. The resistance at this time was measured as the static friction (N). Fig. 7 shows the results of the static friction measurement on the anti-adhesion membranes.

Fig. 7 reveals that anti-adhesion membranes with projections and recesses have a lower static friction than flat anti-adhesion membranes. Accordingly, anti-adhesion membranes with projections and recesses can be passed through a trocar with lower friction than flat anti-adhesion membranes, and therefore can be easily embedded.

### (Experiment 2)

Sodium hyaluronate (Sigma, derived from Streptococcus equi) was dissolved in distilled water to prepare a solution containing 1% by weight of sodium hyaluronate, and carboxymethyl cellulose sodium (Wako Pure Chemical Industries, Ltd., for chemical use) was dissolved in distilled water to prepare a solution containing 0.5% by weight of carboxymethyl cellulose sodium. A 33-mL portion of the mixture of these solutions was cast onto a plastic release sheet with projections and recessed on the surface (size: 10 cm × 10 cm), and air-dried. In this manner, a hyaluronic acid/carboxymethyl cellulose film was obtained.

In the same way, hyaluronic acid/carboxymethyl cellulose films with projections and recesses of a pin spot pattern and flat hyaluronic acid/carboxymethyl cellulose films without projections and recesses having various thicknesses were obtained by using release sheets with an appropriate surface pattern and changing the amount of the hyaluronic acid/carboxymethyl cellulose solution.

A 1% by weight sodium hyaluronate aqueous solution had a viscosity of 89.7 mPa·s, a 0.5% by weight carboxymethyl cellulose sodium had a viscosity of 24.5 mPa·s, and the mixture of these solutions had a viscosity of 137.0 mPa·s.

### (Evaluation)

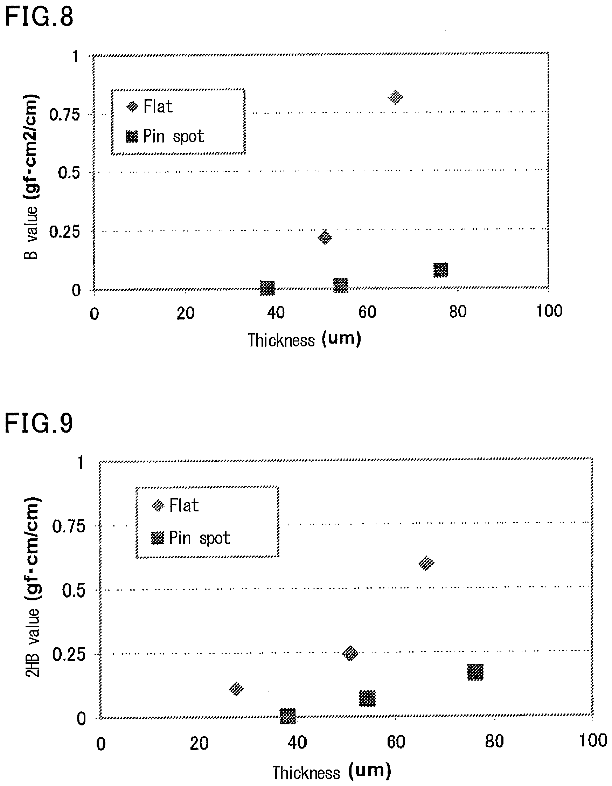

The anti-adhesion membranes were evaluated for bending rigidity and bending hysteresis by the methods described above. Fig. 8 shows the results of the B value measurement on the anti-adhesion membranes, and Fig. 9 shows the results of the 2HB value measurement on the anti-adhesion membranes.

### INDUSTRIAL APPLICABILITY

The present invention provides an anti-adhesion membrane that can be readily rolled up into a thin tube and introduced into the body through a trocar without damage, and has moderate stiffness, and therefore is easy to handle.

## Claims

1. An anti-adhesion membrane comprising a film made of a bioabsorbable material, the film being provided with projections and recesses
wherein the shape of projections and recesses is projections and recesses of a pin spot pattern in which rounded projections and rounded recesses are arranged in a regular pattern, ruminating pattern which refers to a ripple pattern in which continuous projected lines and continuous recessed lines alternately run in parallel, or pyramid pattern in which projections with a multi-sided pyramide shape and recesses with a multi-sided pyramide shape are arranged in a regular pattern.

2. The anti-adhesion membrane according to claim 1,
wherein the film that is made of a bioabsorbable material and provided with projections and recesses comprises crosslinked gelatin.

3. The anti-adhesion membrane according to claim 1,
wherein the film that is made of a bioabsorbable material and provided with projections and recesses comprises hyaluronic acid and carboxymethyl cellulose.

## Patentansprüche

1. Antiadhäsionsmembran, umfassend eine Folie, hergestellt aus einem bioabsorbierbaren Material, wobei die Folie mit Vorsprüngen und Mulden versehen ist,
wobei die Form der Vorsprünge und Mulden Vorsprünge und Mulden eines Nadelpunktmusters sind, wobei gerundete Vorsprünge und gerundete Mulden in einem regelmäßigen Muster, einem Wiederkäumuster (ruminating pattern), welches sich auf ein Wellenmuster bezieht, in welchem hervorspringende Linien und kontinuierlich eingedrückte Linien abwechselnd parallel laufen, oder einem Pyramidenmuster, in welchem Vorsprünge mit einer mehrseitigen Pyramidenform und Mulden mit einer mehrseitigen Pyramidenform in einem regelmäßigen Muster angeordnet sind, angeordnet sind.

2. Antiadhäsionsmembran gemäß Anspruch 1,
wobei die Folie, welche aus einem bioabsorbierbaren Material hergestellt ist und mit Vorsprüngen und Mulden versehen ist, vernetzte Gelatine umfasst.

3. Antiadhäsionsmembran gemäß Anspruch 1,
wobei die Folie, welche aus einem bioabsorbierbaren Material hergestellt ist und mit Vorsprüngen und Mulden versehen ist, Hyaluronsäure und Carboxymethylcellulose umfasst.

## Revendications

1. Membrane anti-adhésion comprenant un film en matériau bioabsorbable, le film étant doté de saillies et d'évidements
dans laquelle la forme des saillies et des évidements est des saillies et des évidements d'un motif de petits points dans lequel des saillies arrondies et des évidements arrondis sont agencés dans un motif régulier, un motif répété qui se réfère à un motif d'ondulation dans lequel des lignes en saillie continues et des lignes évidées continues courent alternativement en parallèle, ou un motif de pyramide dans lequel des saillies de la forme d'une pyramide à faces multiples et des évidements de la forme d'une pyramide à faces multiples sont agencés dans un motif régulier.

2. Membrane anti-adhésion selon la revendication 1,
dans laquelle le film qui est composé d'un matériau bioabsorbable et doté de saillies et d'évidements comprend de la gélatine réticulée.

3. Membrane anti-adhésion selon la revendication 1,
dans laquelle le film qui est composé d'un matériau bioabsorbable et doté de saillies et d'évidements comprend de l'acide hyaluronique et de la carboxyméthylcellulose.
